(19)

Europäisches
Patentamt
European
Patent Office
Office européen
des brevets

(11) **EP 2 698 993 A1**

(12) **EUROPEAN PATENT APPLICATION**
published in accordance with Art. 153(4) EPC

(43) Date of publication:
**19.02.2014 Bulletin 2014/08**

(21) Application number: **12771307.1**

(22) Date of filing: **29.03.2012**

(51) Int Cl.:
**H04N 7/173** [(2011.01)]    **H04L 12/56** [(0000.00)]

(86) International application number:
**PCT/JP2012/058379**

(87) International publication number:
**WO 2012/141014 (18.10.2012 Gazette 2012/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.04.2011 JP 2011087233**

(71) Applicant: **Sony Corporation
Tokyo 108-0075 (JP)**

(72) Inventor: **RANATUNGA, Vijitha Sanjeewa
Tokyo 108-0075 (JP)**

(74) Representative: **Robinson, Nigel Alexander Julian
D Young & Co LLP
120 Holborn
London EC1N 2DY (GB)**

(54) **DISPLAY CONTROL DEVICE, DISPLAY CONTROL METHOD, AND PROGRAM**

(57)    The present technique relates to a display control apparatus, a display control method, and a program capable of suppressing an image distortion and the like generated when an image transmitted from a data transmission line is displayed with a low delay. A first computation unit computes an allowable delay time indicating a delay time allowed when it is difficult to terminate the writing with a predetermined elapsed time from a write start time at which the image starts to be written. A second computation unit computes a necessary duration necessary at least until a display timing, at which the image starts to be displayed, from the write start time of the image written with a delay time equal to or shorter than the allowable delay time. A comparison unit compares the allowable delay time and the necessary duration. A display adjustment unit adjusts the display timing based on a result of the comparison in the comparison unit. A display control unit displays the image in synchronization with the adjusted display timing. This technique can be applied to, for example, a display device that displays transmitted image data with a low delay.

FIG. 7

EP 2 698 993 A1

**Description**

TECHNICAL FIELD

This disclosure relates to a display control apparatus, a display control method, and a program, and more particularly, to a display control apparatus, a display control method, and a program capable of suppressing, for example, an image distortion generated when an image transmitted from a data transmission line such as a network is displayed with a low delay.

BACKGROUND ART

In recent years, telesurgery has been proposed, in which a surgical operation for a patient is performed by operating a robot arm in a remote place. In this telesurgery, an operating surgeon operates a robot arm while the operating surgeon watches a moving picture obtained by shooting a surgical operation state. Therefore, it is desirable to transmit the moving picture with a low delay equal to or lower than several frames or fields (nearly in a real-timely manner).
In this regard, a coding technique has been proposed, in which several lines of each picture included in the moving picture are set as block data, and a wavelet transform coding (compression) is performed for each block data (for example, refer to Patent Document 1)
In this coding technique, a transmitter initiates the coding without waiting for overall input for each block data in a picture and transmits the resulting coded data, and a receiver initiates decoding (decompression) before overall coded data are received from the transmitter.
For this reason, the receiver can make the picture in a displayable state by decoding the coded data until a predetermined display timing for displaying the picture. Therefore, receiver can display the picture on a monitor in synchronization with the display timing.

CITATION LIST

PATENT DOCUMENT

    Patent Document 1: Japanese Patent Application Laid-Open No. 2007-311924

SUMMARY OF THE INVENTION

PROBLEMS TO BE SOLVED BY THE INVENTION

However, for example, in a case where congestion occurs in a data transmission line such as a network, it may be difficult for a transmitter to transmit a moving picture with a low delay equal to or lower than several frames.
In this case, it is difficult for a receiver to make a picture in a displayable state until a predetermined display timing. The picture that is not in the displayable state until the display timing is skipped without being displayed on a monitor. Therefore, this may generate a distortion and the like in an image displayed on the monitor.
In view of such a situation, this disclosure has been made to suppress an image distortion generated when an image transmitted from a data transmission line is displayed with a low delay.

SOLUTIONS TO PROBLEMS

According to an aspect of this disclosure, there is provided a display control apparatus including: a receive unit that receives an image; a write unit that writes the image in a holding unit that temporarily holds the received image; a first computation unit that computes an allowable delay time indicating a delay time allowed when it is difficult to terminate the writing within a predetermined elapsed time from a write start time at which the image starts to be written; a second computation unit that computes a necessary duration that is necessary at least until a display timing, at which the image starts to be displayed, from the write start time of the image written within a delay time equal to or shorter than the allowable delay time; a comparison unit that compares the allowable delay time and the necessary duration; a display adjustment unit that adjusts the display timing based on a result of the comparison in the comparison unit; and a display control unit that displays the image in synchronization with the adjusted display timing.
The display control apparatus may further include a first measurement unit that measures a write time necessary to write the image, and the first computation unit may compute the allowable delay time based on a distribution of the write time.

The display control apparatus may further include a second measurement unit that measures a write preparation duration necessary to start the write the image, and the second measurement unit may compute the necessary duration based on the distribution of the write preparation duration.

In the display control apparatus described above, the display control may terminate display of the image at a predetermined time from the display timing in synchronization with the adjusted display timing, and the display adjustment unit may adjust the display timing to shorten the necessary duration as long as a condition that the necessary duration is longer than the allowable delay time is satisfied in a case where the necessary duration is longer than the allowable delay time as a result of the comparison.

In the display control apparatus described above, wherein the display adjustment unit may adjust the display timing to obtain the necessary duration longer than the allowable delay time in a case where the necessary duration is not longer than the allowable delay time as a result of the comparison.

In the display control apparatus described above, the write unit may start to write the image after the first display timing in a case where the necessary duration is not longer than the delay time as a result of the comparison, and the display control unit may display the image in synchronization with a second display timing later than the first display timing.

In the display control apparatus described above, the display control unit may display the image written with a delay time equal to or shorter than the allowable delay time in synchronization with the adjusted display timing.

According to another aspect of this disclosure, there is provided a display control method of a display control apparatus for displaying an image, the display control method including: a receiving step of receiving the image; a writing step of writing the image to a holding unit that temporarily holds the received image; a first computing step of computing an allowable delay time indicating a delay time allowable when it is difficult to terminate the writing within a predetermined elapsed time from a write start time at which the image starts to be written; a second computing step of computing a necessary duration that is necessary at least until a display timing, at which the image starts to be displayed, from the write start time of the image written within a delay time equal to or shorter than the allowable delay time; a comparing step of comparing the allowable delay time and the necessary duration; a display adjusting step of adjusting the display timing based on a result of the comparison in the comparing step; and a display controlling step of displaying the image in synchronization with the adjusted display timing.

According to still another aspect of this disclosure, there is provided a program causing a computer to serve as: a receiving control unit that receives an image; a write unit that writes the image in a holding unit that temporarily holds the received image; a first computation unit that computes an allowable delay time indicating a delay time allowed when it is difficult to terminate the writing within a predetermined elapsed time from a write start time at which the image starts to be written; a second computation unit that computes a necessary duration that is necessary at least until a display timing, at which the image starts to be displayed, from the write start time of the image written within a delay time equal to or shorter than the allowable delay time; a comparison unit that compares the allowable delay time and the necessary duration; a display adjustment unit that adjusts the display timing based on a result of the comparison in the comparison unit; and a display control unit that displays the image in synchronization with the adjusted display timing.

According to further another aspect of this disclosure, an image is received, the image is written to a holding unit that temporarily holds the received image, an allowable delay time indicating a delay time allowed when it is difficult to terminate the writing within a predetermined elapsed time from the write start time at which the image starts to be written is computed, a necessary duration necessary at least until a display timing, at which the image starts to be displayed, from the write start time of the image written with a delay time equal to or shorten than the allowable delay time is computed, the allowable delay time and the necessary duration are compared, the display timing is adjusted based on a result of the comparison, the image is displayed in synchronization with the adjusted display timing.

EFFECTS OF THE INVENTION

According to this disclosure, it is possible to suppress an image distortion and the like generated when an image transmitted from a data transmission line is displayed with a low delay.

BRIEF DESCRIPTION OF DRAWINGS

Fig. 1 is a block diagram illustrating an exemplary configuration of a transceiver system 1 according to this disclosure.
Fig. 2 is a diagram illustrating an example of displaying an image in synchronization with a display timing.
Fig. 3 is a diagram illustrating an exemplary method of measuring write information.
Fig. 4 is a diagram illustrating an exemplary distribution of a write preparation duration.
Fig. 5 is a diagram illustrating an exemplary distribution of a buffer write duration.

Fig. 6 is a diagram illustrating an exemplary method of computing a necessary duration and an allowable delay time.

Fig. 7 is a first diagram illustrating a method of adjusting the display timing.

Fig. 8 is a second diagram illustrating a method of adjusting the display timing.

Fig. 9 is a first diagram illustrating an exemplary case where the transmission duration changes.

Fig. 10 is a second diagram illustrating an exemplary case where the transmission duration changes.

Fig. 11 is a flowchart illustrating a display control process.

Fig. 12 is a flowchart illustrating a display timing adjustment process.

Fig. 13 is a diagram illustrating an exemplary case where a timing for writing an image is delayed.

Fig. 14 is a diagram illustrating an exemplary method of measuring write information of an odd-numbered field image.

Fig. 15 is a diagram illustrating an exemplary method of measuring write information of a progressive image.

Fig. 16 is a block diagram illustrating an exemplary computer configuration.

MODE FOR CARRYING OUT THE INVENTION

Hereinafter, an embodiment of this disclosure (hereinafter, referred to as an embodiment) will be described. Description will be made in the following sequence.

1. First Embodiment: an example of suppressing an image distortion and the like by adjusting a display timing Vblank

2. Second Embodiment: an example of suppressing an image distortion and the like without adjusting a display timing Vblank

3. Modifications

1. First Embodiment

<Exemplary Configuration of Transceiver System 1>

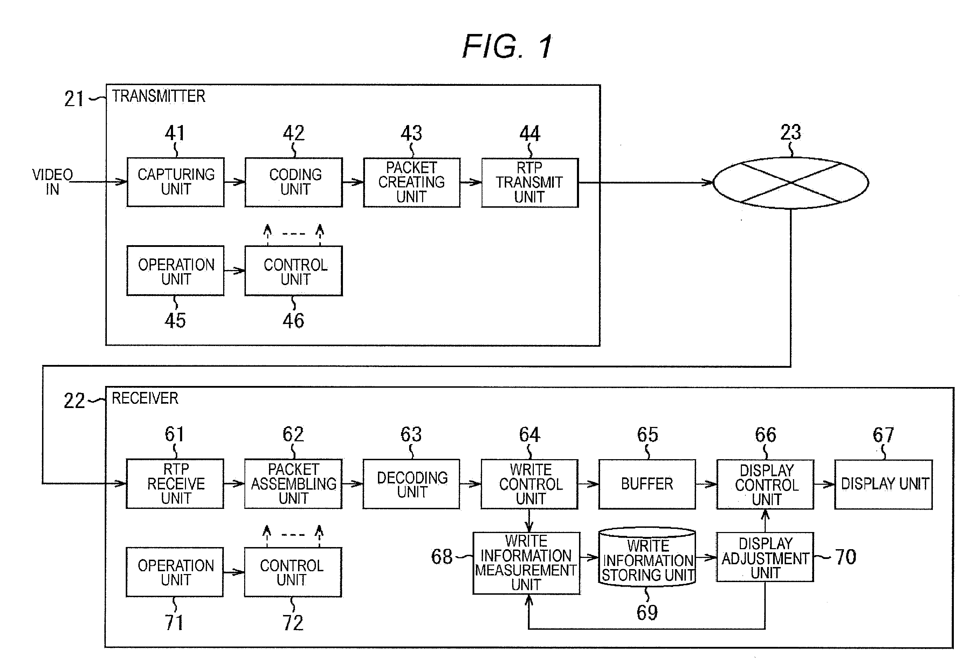

Fig. 1 illustrates a transceiver system 1 according to this disclosure.

The transceiver system 1 includes a transmitter 21, a receiver 22, and a network 23 such as the Internet.

In the receiver 22 side of the transceiver system 1, an image is displayed with a low delay without generating a distortion and the like in the image transmitted from the transmitter 21, for example, by adjusting a display timing Vblank for displaying the image.

<Exemplary Configuration of Transmitter 21>

The transmitter 21 includes a capturing unit 41, a coding unit 42, a packet creating unit 43, a real-time transport protocol (RTP) transmit unit 44, an operation unit 45, and a control unit 46.

The capturing unit 41 obtains (captures) image data (corresponding to VIDEO IN) input from the outside and supplies the image data to the coding unit 42.

The coding unit 42 performs a coding process for coding the image data from the capturing unit 41 and supplies the coded data obtained through the coding process to the packet creating unit 43.

As the coding process, a wavelet coding process may be employed, in which image data are compressed, for example, by performing a wavelet transform-based coding.

The packet creating unit 43 packetizes (or converts) the coded data from the coding unit 42 into a plurality of RTP packets and outputs the RTP packets to the RTP transmit unit 44. The RTP packet refers to a packet based on a RTP standard specified in the Internet engineering task force (IETF) request for comments (RFC) 3550.

The RTP transmit unit 44 adds, for example, a transmission time point, as a time stamp, indicating when the RTP packet is transmitted to the receiver 22, to the RTP packet from the packet creating unit 43 according to the RTP. In addition, the RTP transmit unit 44 transmits the RTP packet having the added time stamp to the receiver 22 via the network 23.

The operation unit 45 is an operation button and the like manipulated by a user. The operation unit 45 supplies an operation signal corresponding to user's operation to the control unit 46.

The control unit 46 controls each of the capturing unit 41, the coding unit 42, the packet creating unit 43, and the RTP transmit unit 44, for example, based on the operation signal from the operation unit 45.

<Exemplary Configuration of Receiver 22>

The receiver 22 includes a RTP receive unit 61, a packet assembling unit 62, a decoding unit 63, a write control unit 64, a buffer 65, a display control unit 66, a display unit 67, a write information measurement unit 68, a write information storing unit 69, a display adjustment unit 70, an operation unit 71, and a control unit 72.

The RTP receive unit 61 receives the RTP packet transmitted from the RTP transmit unit 44 via the network 23 and supplies the RTP packet to the packet assembling unit 62.

The packet assembling unit 62 creates coded data as a decoding target by assembling the RTP packets from the RTP receive unit 61 and supplies the coded data to the decoding unit 63.

The decoding unit 63 performs a decoding process corresponding to the coding process of the coding unit 42 for the coded data from the packet assembling unit 62 and supplies the resulting image data to the write control unit 64. Here, the decoding process may include, for example, an inverse wavelet transform for decompression.

The write control unit 64 supplies the image data from the decoding unit 63 to the buffer 65, and the image data is stored in (written to) the buffer 65. In addition, the write control unit 64 supplies the write information measurement unit 68 with a write start time Ts indicating time when each image data starts to be written to the buffer 65 and a write end time Te indicating time when the writing to the buffer 65 is terminated.

The buffer 65 temporarily holds the image data from the write control unit 64.

The display control unit 66 reads the image data from the buffer 65 and supplies the image data to the display unit 67. In addition, the display control unit 66 causes the display unit 67 to display the image data read from the buffer 65 in synchronization with the display timing Vblank from the display adjustment unit 70.

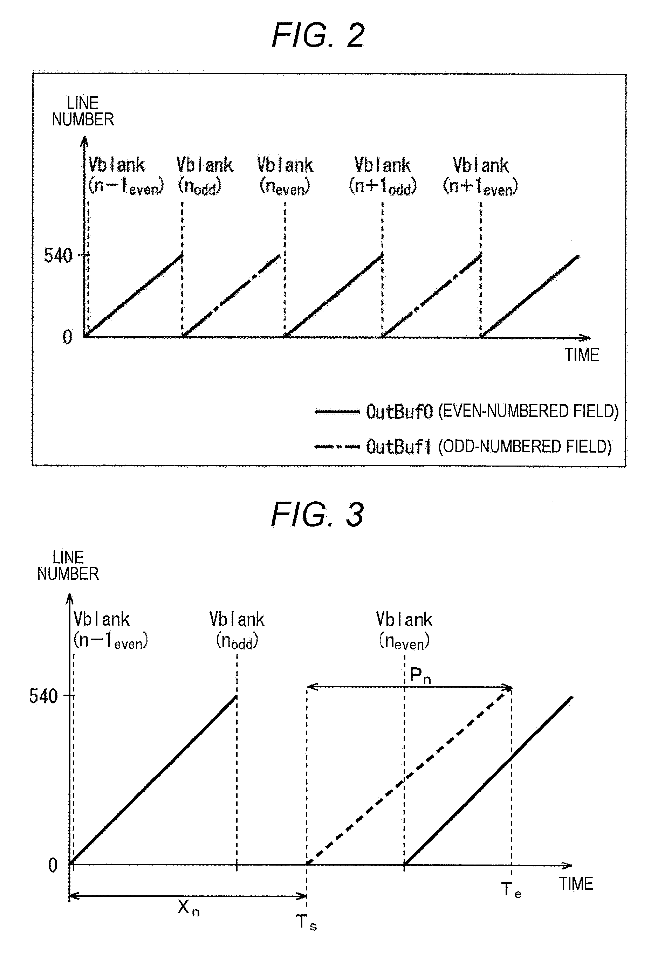

Next, Fig. 2 illustrates an exemplary case where the display control unit 66 displays each image data at a display timing Vblank from the display adjustment unit 66.

It is assumed that the image data n includes an odd-numbered field image $n_{odd}$ corresponding to odd-numbered line out of lines of the image data n and an even-numbered field image $n_{even}$ corresponding to even-numbered line out of lines of the image data n.

In Fig. 2, the abscissa denotes time, and the ordinate denotes a line number of the line displayed at the corresponding time point. Here, it is assumed that the image data n has, for example 1080 lines, and each of the odd-numbered field image $n_{odd}$ and the even-numbered field image $n_{even}$ has 540 lines.

The display timing $Vblank(n_{odd})$ refers to a time point when the odd-numbered field image $n_{odd}$ starts to be displayed. Similarly, the display timing $Vblank(n_{even})$ refers to a time point when the even-numbered field image $n_{even}$ starts to be displayed.

The display control unit 66 reads the odd-numbered field image $n_{odd}$ from the buffer 65 and displays the odd-numbered field image $n_{odd}$ on the display unit 67 in synchronization with the display timing $Vblank(n_{odd})$ from the display adjustment unit 70. In addition, the display control unit 66 reads the even-numbered field image $n_{even}$ from the buffer 65 in synchronization with the display timing $Vblank(n_{even})$ from the display adjustment unit 70 and displays the even-numbered field image $n_{even}$ on the display unit 67.

The display unit 67 displays each image data n, for example, in an interlace mode. That is, the display unit 67 alternately displays the odd-numbered field image $n_{odd}$ and the even-numbered field image $n_{even}$ under control of the display control unit 66. The display unit 67 may be included in the receiver 22 as an element as illustrated in Fig. 1 or may be provided independently from the receiver 22. In this case, the display unit 67 provided outside the receiver 22 is connected to the display control unit 66 of the receiver 22, for example, through a cable and the like.

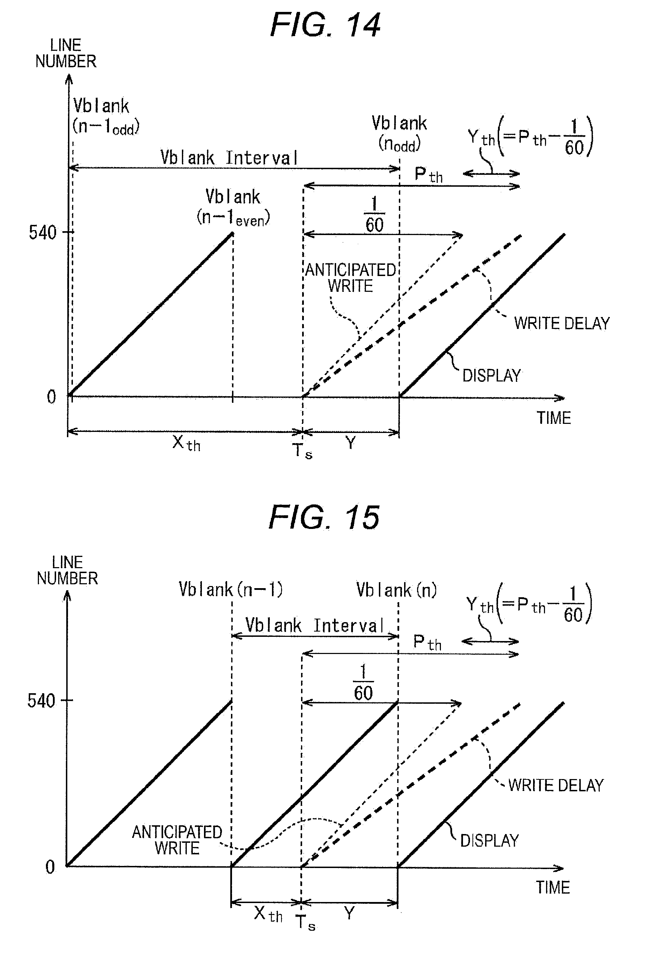

The write information measurement unit 68 measures write information of the even-numbered field image $n_{even}$ written to the buffer 65. In addition, the write information measurement unit 68 may measure the write information of the odd-numbered field image $n_{odd}$ written to the buffer 65 instead of the write information of the even-numbered field image $n_{even}$ or along with the write information of the even-numbered field image $n_{even}$. This will be described below with reference to Fig. 14.

For example, in a case where the display unit 67 displays each image data n in a progressive mode, each image data n is converted into a progressive image, and the write information measurement unit 68 measures the write information of the progressive image. This will be described below with reference to Fig. 15.

Hereinafter, description will be made by assuming that the write information measurement unit 68 measures the write information of the even-numbered field image $n_{even}$ written to the buffer 65.

The write information measurement unit 68 measures, for example, a write preparation duration Xn necessary until the even-numbered field image $n_{even}$ starts to be written and a buffer write duration Pn necessary to write the even-numbered field image $n_{even}$ as the write information of the even-numbered field image $n_{even}$ written to the buffer 65.

Next, Fig. 3 illustrates an exemplary case where the write information measurement unit 68 measures the write preparation duration Xn and the buffer write duration Pn of the even-numbered field image $n_{even}$.

Here, in Fig. 3, the write start time Ts refers to a time point when the even-numbered field image $n_{even}$ starts to be written to the buffer 65. In addition, the write end time Te refers to a time point when the writing of the even-numbered field image $n_{even}$ to the buffer 65 is terminated.

In addition, the write information measurement unit 68 is supplied with the write start time Ts and the write end time Te of the even-numbered field image $n_{even}$ from the write control unit 64 and the display timings $Vblank(n-1_{even})$ and $Vblank(n_{even})$ from the display adjustment unit 70.

The write information measurement unit 68 measures the write preparation duration $Xn(=Vblank(n-1_{even})-Ts)$ based on the write start time Ts from the write control unit 64 and the display timing $Vblank(n-1_{even})$ from the display adjustment unit 70 as illustrated in Fig. 3.

For example, the write information measurement unit 68 measures the buffer write duration $Pn(=Te-Ts)$ based on the write end time Te and the write start time Ts from the write control unit 64 as illustrated in Fig. 3.

The write information measurement unit 68 supplies the write preparation duration Xn and the buffer write duration Pn measured for each even-numbered field image $n_{even}$ to the write information storing unit 69 to store the write preparation duration Xn and the buffer write duration Pn in the write information storing unit 69.

Returning to Fig. 1, the write preparation duration Xn and the buffer write duration Pn from the write information measurement unit 68 are stored in the write information storing unit 69 as the write information.

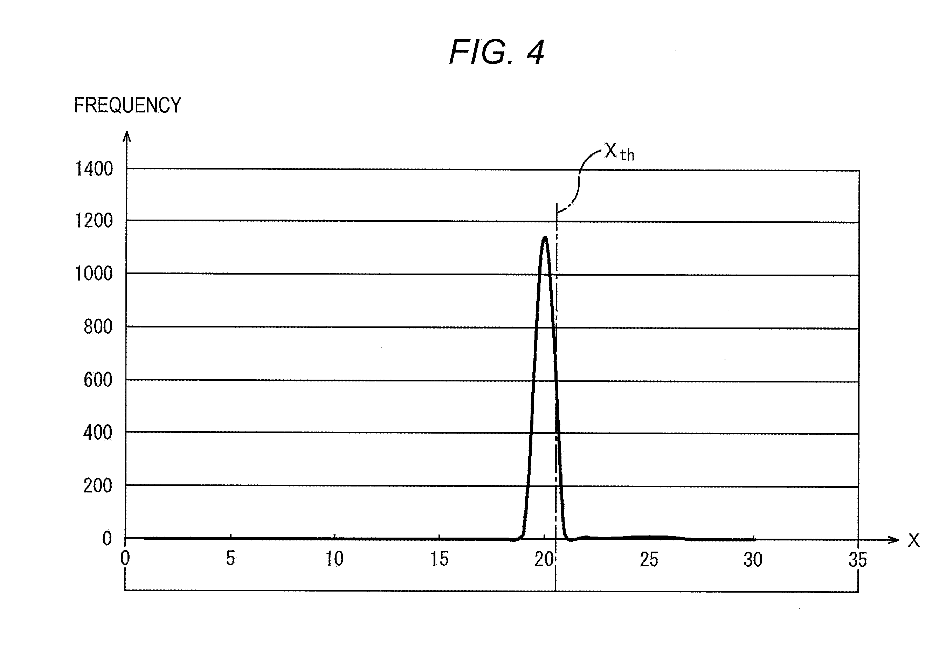

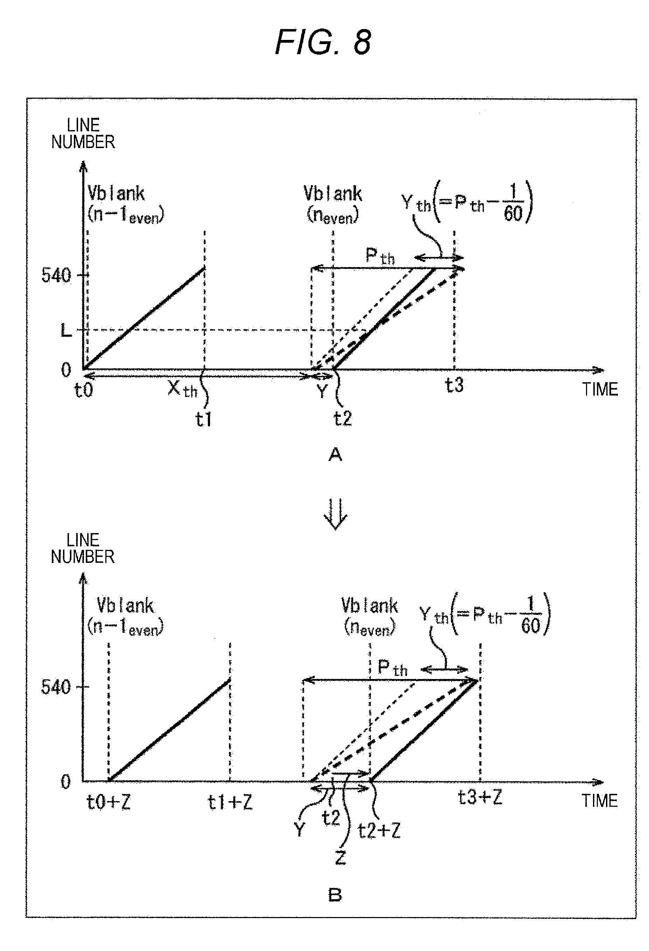

The display adjustment unit 70 performs a display timing adjustment process for adjusting the display timing Vblank based on the write information stored in the write information storing unit 69. The display timing adjustment process will be described in detail with reference to Figs. 4 and 8.

The display adjustment unit 70 supplies the display timing Vblank adjusted through the display timing adjustment process to the display control unit 66 and the write information measurement unit 68.

The operation unit 71 includes an operation button manipulated by a user and supplies an operation signal corresponding to the user's operation to the control unit 72.

The control unit 72 controls each of the RTP receive unit 61, the packet assembling unit 62, the decoding unit 63, the write control unit 64, the display control unit 66, the write information measurement unit 68, and the display adjustment unit 70 based on the operation signal from the operation unit 71.

<Details of Display Timing Adjustment Process>

Next, a display timing adjustment process performed by the display adjustment unit 70 will be described with reference to Figs. 4 to 8.

Fig. 4 illustrates an exemplary distribution of the write preparation durations Xn stored in the write information storing unit 69.

In Fig. 4, the abscissa denotes the write preparation duration Xn, and the ordinate denotes a frequency of the write preparation durations Xn.

The display adjustment unit 70 creates X-distribution information indicating a distribution of the write preparation durations Xn of Fig. 4 based on the write preparation durations Xn stored in the write information storing unit 69.

In addition, the display adjustment unit 70 computes a threshold value Xth for distinguishing a higher rank $\alpha\%$ (for example, $\alpha=95$) of the write preparation durations Xn of and a lower rank $(100-\alpha)\%$ of the remaining write preparation durations Xn obtained by sorting the write preparation durations Xn in an ascending order based on the X-distribution information of the write preparation duration Xn.

Here, the threshold value Xth is set to a value equal to or greater than the maximum value of a higher rank $\alpha\%$ (for example, $\alpha=95$) of the write preparation durations Xn and smaller than the minimum value of a lower rank $(100-\alpha)\%$ of the write preparation durations Xn obtained by sorting the write preparation durations Xn in an ascending order. The value $\alpha$ is predetermined, for example, through user's operation.

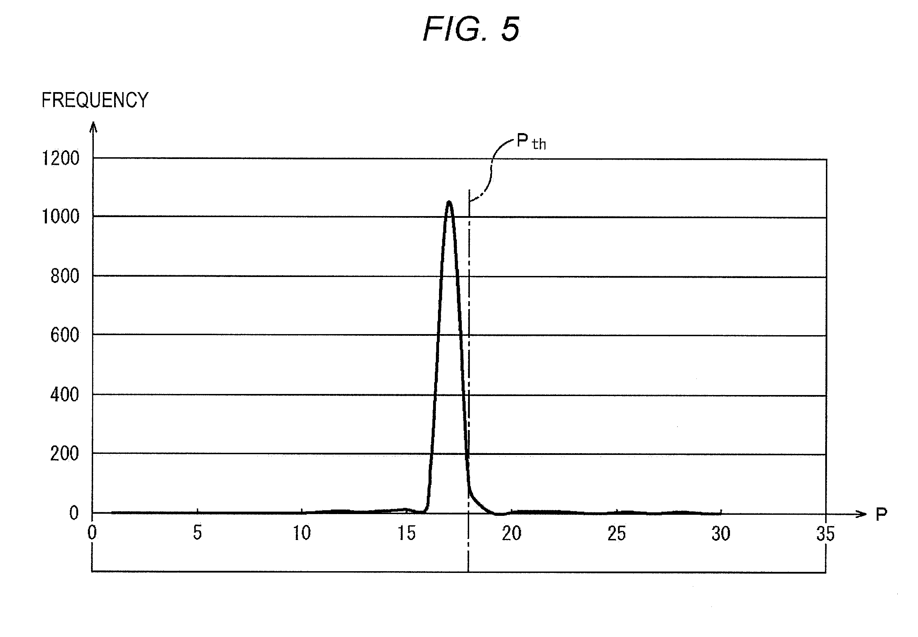

Next, Fig. 5 illustrates an exemplary distribution of the buffer write durations Pn stored in the write information storing unit 69.

In Fig. 5, the abscissa denotes the buffer write duration Pn, and the ordinate denotes a frequency of the buffer write durations Pn.

The display adjustment unit 70 creates P-distribution information indicating a distribution of the buffer write durations Pn of Fig. 5 based on a plurality of buffer write durations Pn stored in the write information storing unit 69.

The display adjustment unit 70 computes a threshold value Pth for distinguishing a higher rank $\alpha\%$ of the buffer write durations Pn and a lower rank $(100-\alpha)\%$ of the buffer write durations Pn obtained by sorting the buffer write durations Pn in an ascending order based on the P-distribution information of the buffer write durations Pn.

Here, the threshold value Pth is set to a value equal to or greater than the maximum value of a higher rank $\alpha\%$ of the buffer write durations Pn and smaller than the minimum value of a lower rank $(100-\alpha)\%$ of the buffer write duration Pn obtained by sorting the buffer write durations Pn in an ascending order.

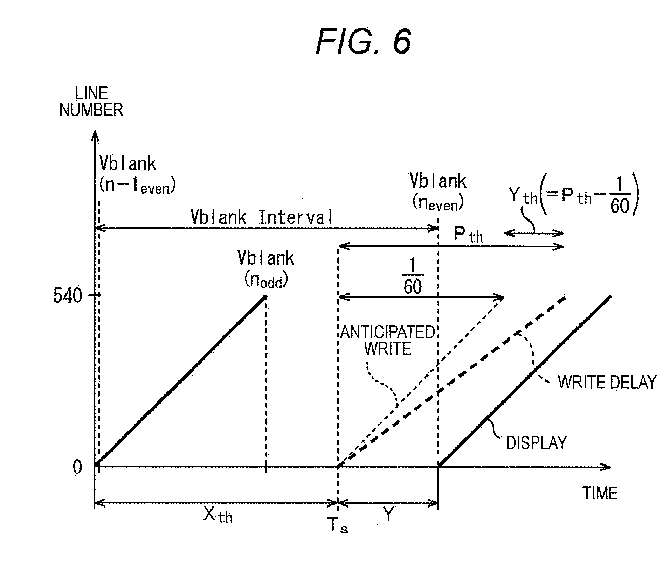

Next, Fig. 6 illustrates an exemplary case where the display adjustment unit 70 computes an allowable delay time Yth based on the threshold value Pth and computes a necessary duration Y based on the threshold value Xth.

The display adjustment unit 70 computes the allowable delay time Yth based on the threshold value Pth computed from the P-distribution information of the buffer write durations Pn using Equation (1) as illustrated in Fig. 6.

$$Yth = Pth - (1/60) \quad \dots \quad (1)$$

Here, the allowable delay time Yth refers to a delay time when it is impossible to terminate the writing within a predetermined anticipated write time 1/60 from the write start time Ts at which the even-numbered field image $n_{even}$ starts to be written. The allowable delay time Yth is a delay time allowed to perform display with a possibility of at least $\alpha\%$ without generating a distortion in the even-numbered field image $n_{even}$.

The anticipated write time 1/60 refers to a write time of the even-numbered field image $n_{even}$ (or odd-numbered field image $n_{odd}$) anticipated in a case where no delay is generated in the data writing in the write control unit 64.

The anticipated write time 1/60 is determined, for example, depending on time necessary to display the even-numbered field image $n_{even}$ (or odd-numbered field image $n_{odd}$). In this case, the display unit 67 displays each of the odd-numbered field image and the even-numbered field image with a display time 1/60. Therefore, for example, the anticipated write time is also set to 1/60 in this manner. In addition, in a case where the display unit 67 displays each the odd-numbered field image and even-numbered field image with a display time 1/m, for example, the anticipated write time is set to 1/m.

For example, the display adjustment unit 70 computes a necessary duration Y of the even-numbered field image $n_{even}$ received with a possibility of $\alpha\%$ based on the threshold value Xth computed from X-distribution information of the write preparation duration Xn, that is, a necessary duration Y of the even-numbered image $n_{even}$ written with a delay time equal to or shorter than the allowable delay time Yth.

Here, the necessary duration Y refers to time necessary at least from the write start time Ts to the display timing ($n_{even}$) in the even-numbered field image $n_{even}$ written to the buffer 65 with a delay time equal to or shorter than the allowable delay time Yth.

For example, the display adjustment unit 70 computes the necessary duration Y based on the display interval Vblank_Interval between the display timing Vblank ($n-1_{even}$) and the display timing ($n_{even}$), and the threshold value Xth using the following Equation (2) as illustrated in Fig. 6.

$$Y = Vblank\_Interval - Xth \quad \dots \quad (2)$$

The display adjustment unit 70 determines whether or not display can be performed with a possibility of at least $\alpha\%$ without generating a distortion and the like in the even-numbered field image $n_{even}$ based on whether or not the computed necessary duration Y satisfies the following Equation (3). In addition, the display adjustment unit 70 adjusts the display timing Vblank based on the determination result.

$$Y > Yth \quad \dots \quad (3)$$

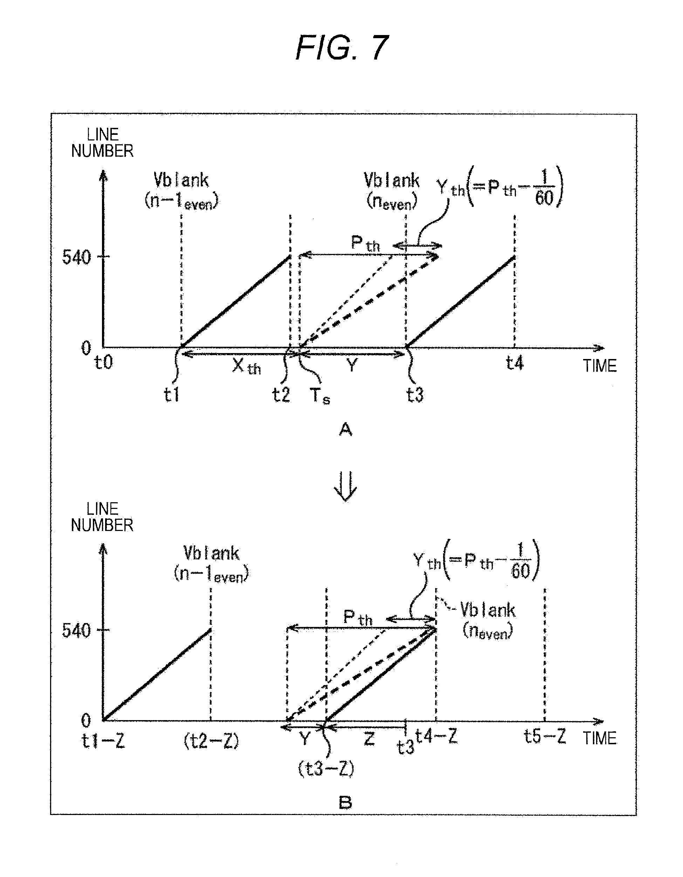

In a case where the Equation (3) is satisfied, the line(i) corresponding to the line number i of the even-numbered field image $n_{even}$ is written to the buffer 65 before the display timing of the line(i) as illustrated in Fig. 7A. In this case, the display adjustment unit 70 adjusts each display timing Vblank to advance by time Z as illustrated in Fig. 7B to infinitely decrease the necessary duration Y as long as the Equation (3) is satisfied.

If the Equation (3) is not satisfied, out of the line(i) of the line number i in the even-numbered field image $n_{even}$, for example, the line (i) ($i \geq L$) following the line number L is written to the buffer 65 after the display timing of the line(i) ($i \geq L$) as illustrated in Fig. 8A. In this case, the display adjustment unit 70 adjusts the display timing Vblank to delay by time Z as illustrated in Fig. 8B to increase the necessary duration Y to satisfy the Equation (3).

The display adjustment unit 70 appropriately performs the display timing adjustment process also after the display timing Vblank. This is because the transmission duration of the image data changes depending on a congestion situation of the network 23 and the like, and the state of Fig. 7A or 8A may occur even in the adjusted display timing Vblank.

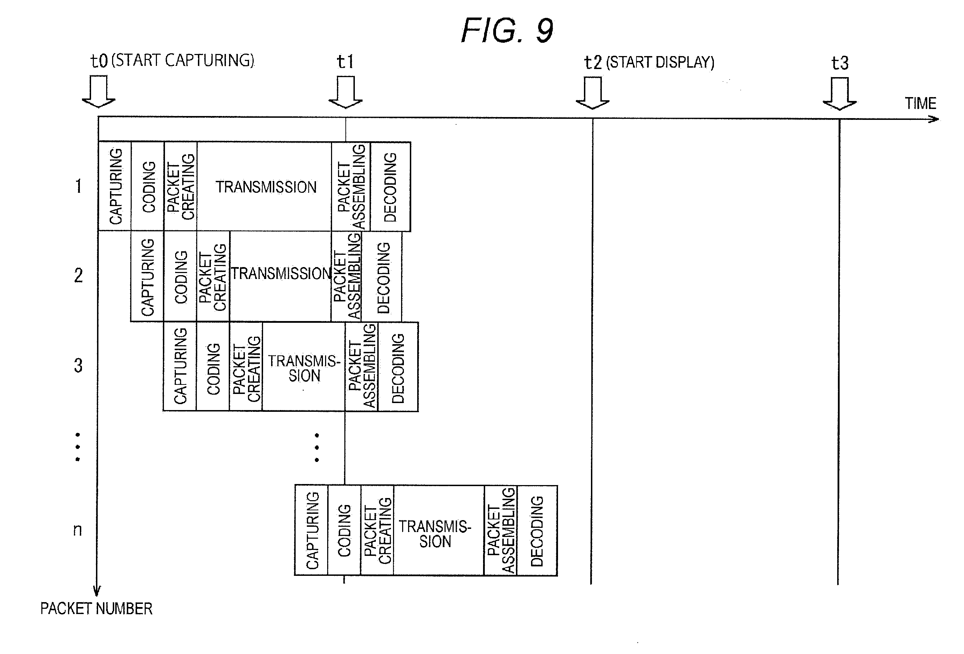

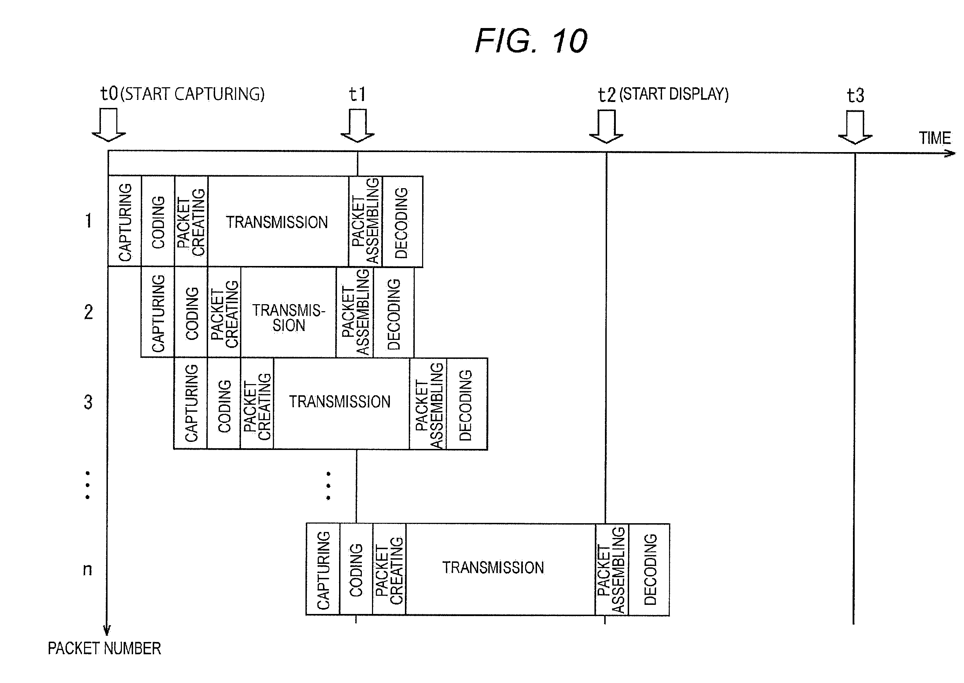

Next, Figs. 9 and 10 illustrate an exemplary case where the transmission duration changes depending on a congestion situation of the network 23 and the like.

In Figs. 9 and 10, the ordinate denotes a packet number of each RTP packet of the image data n. In addition, in the abscissa, durations necessary to perform a capturing process of the capturing unit 41, a coding process of coding unit 42, a packet creating process of the packet creating unit 43, a RTP packet transmission process between the RTP transmit unit 44 and the RTP receive unit 61 via the network 23, a packet assembling process of the packet assembling unit 62, and a decoding process of the decoding unit 63 for the data of the image data n are illustrated.

As illustrated in Figs. 9 and 10, the time necessary for the transmission process changes depending on a situation of the network 23. That is, in a case where congestion of the network 23 is relatively insignificant, the time necessary for the transmission process is shortened as illustrated in Fig. 9. In a case where congestion of the network 23 is relatively significant, the duration necessary for the transmission process is lengthened as illustrated in Fig. 10.

Therefore, the time necessary for the transmission process, that is, the transmission duration changes depending on a congestion situation of the network 23 and the like. Accordingly, the state illustrated in Fig. 7A or 8A may occur. Therefore, it is necessary for the display adjustment unit 70 to appropriately perform the display timing adjustment process.

<Description of Operations in Receiver 22>

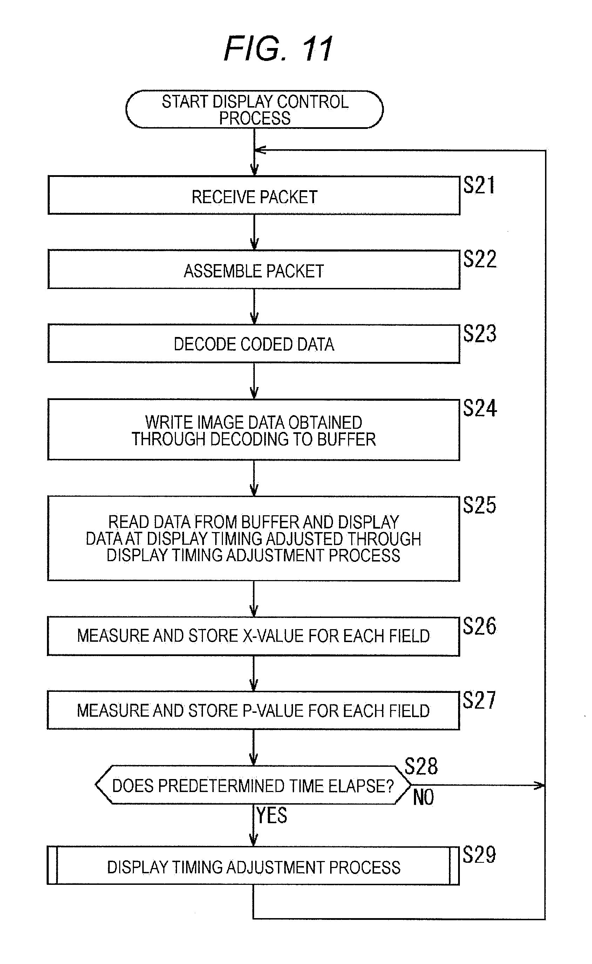

Next, a display control process performed by the receiver 22 will be described with reference to the flowchart of Fig. 11.

In step S21, the RTP receive unit 61 receives a RTP packet transmitted from the RTP transmit unit 44 via the network 23 and supplies the RTP packet to the packet assembling unit 62.

In step S22, the packet assembling unit 62 assembles the RTP packets from the RTP receive unit 61 to generate coded data as a decoding target and supplies the coded data to the decoding unit 63.

In step S23, the decoding unit 63 performs a decoding process corresponding to the coding process of the coding unit 42 for the coded data from the packet assembling unit 62 and supplies the resulting image data to the write control unit 64. Here, the decoding process may include, for example, an inverse wavelet transform for decompression.

In step S24, the write control unit 64 supplies the image data (including the odd-numbered field image and the even-numbered field image) from the decoding unit 63 to the buffer 65, the image data are stored in (or written to) the buffer 65. In addition, the write control unit 64 supplies the write information measurement unit 68 with the write start time Ts at which the writing to the buffer 65 starts and the write end time Te at which the writing to the buffer 65 terminates for each even-numbered field image $n_{even}$.

In step S25, the display control unit 66 reads the image data including the odd-numbered field image and the even-numbered field image from the buffer 65 and displays the image data on the display unit 67 in synchronization with the display timing Vblank from the display adjustment unit 70.

In step S26, the write information measurement unit 68 measures the write preparation duration Xn of the even-numbered field image $n_{even}$ based on the write start time Ts from the write control unit 64 and the display timing Vblank from the display adjustment unit 70 and supplies the write preparation duration Xn to the write information storing unit 69. Then, the write preparation duration Xn is stored in the write information storing unit 69.

In step S27, the write information measurement unit 68 measures the buffer write duration Pn of the even-numbered field image $n_{even}$ based on write start time Ts and the write end time Te from the write control unit 64 and supplies the buffer write duration Pn to the write information storing unit 69. Then, the buffer write duration Pn is stored in the write information storing unit 69.

In step S28, the display adjustment unit 70 determines whether or not a time count counted by a built-in time count unit (not illustrated) exceeds a predetermined time. If it is determined that the time count does not exceed the predetermined time, the process returns to step S21 to repeat the same operation.

If the display adjustment unit 70 determines that the time count exceeds the predetermined time in step S28, the process advances to step S29, in which a display timing adjustment process is performed to adjust the display timing Vblank based on the write preparation duration Xn and the buffer write duration Pn stored in the write information storing unit 69.

In step S29, the display adjustment unit 70 deletes the write preparation duration Xn and the buffer write duration Pn stored in the write information storing unit 69 after the display timing adjustment process. In addition, the display adjustment unit 70 resets the time count of the built-in time count unit and restarts the time counting. Then, the process returns to step S21 to repeat the same operation.

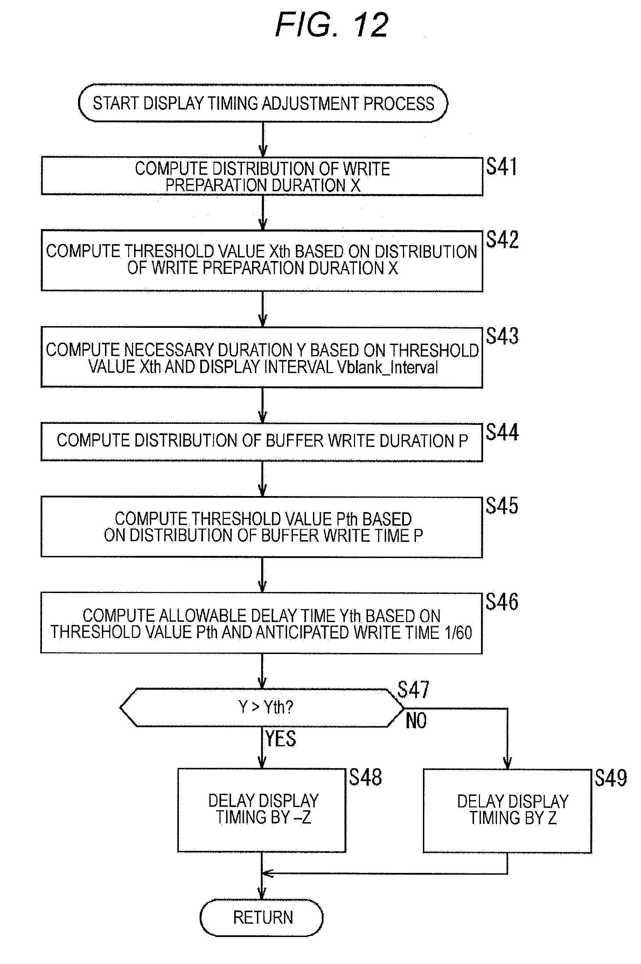

Next, the display timing adjustment process in step S29 of Fig. 11 will be described in detail with reference to the flowchart of Fig. 12.

In step S41, the display adjustment unit 70 reads the write preparation duration Xn from the write information storing unit 69 and computes the X-distribution information indicating a distribution of the write preparation durations Xn.

In step S42, the display adjustment unit 70 computes the threshold value Xth based on the X-distribution information computed in step S41.

In step S43, the display adjustment unit 70 computes the necessary duration Y based on the display interval Vblank_Interval and the threshold value Xth using the Equation (2).

In step S44, the display adjustment unit 70 reads the buffer write duration Pn from the write information storing unit 69 and computes the P-distribution information indicating a distribution of the buffer write durations Pn.

In step S45, the display adjustment unit 70 computes the threshold value Pth based on the P-distribution information computed in step S41.

In step S46, the display adjustment unit 70 computes the allowable delay time Yth based on the anticipated write time 1/60 and the threshold value Pth computed in step S45 using the Equation (1).

In step S47, the display adjustment unit 70 determines whether or not the Equation (3) is satisfied based on the necessary duration Y computed in step S43 and the allowable delay time Yth computed in step S46. If it is determined that the Equation (3) is satisfied, the process advances to step S48.

In step S48, the display adjustment unit 70 adjusts the display timing Vblank to advance by time Z as illustrated in Fig. 7 and supplies the adjusted display timing Vblank to the display control unit 66.

In step S47, if the display adjustment unit 70 determines that the Equation (3) is not satisfied based on the necessary duration Y computed in step S43 and the allowable delay time Yth computed in step S46, the process advances to step S49.

In step S49, the display adjustment unit 70 adjusts the display timing Vblank to delay by time Z as illustrated in Fig. 8 and supplies the adjusted display timing Vblank to the display control unit 66.

After the process in step S48 or S49 is terminated as described above, the process returns to step S29 of Fig. 11, and the subsequent processes are performed.

As described above, in the display control process, the display timing Vblank is adjusted to satisfy the Equation (3), for example, depending on the write information of the even-numbered field image $n_{even}$. Therefore, it is possible to suppress an image distortion that may be generated when a part of the image is skipped. That is, the display control unit 66 can display the even-numbered field image $n_{even}$ and the odd-numbered field image $n_{odd}$ written to the buffer 65 with a delay time equal to or shorter than the allowable delay time Yth without generating an image distortion and the like in synchronization with the display timing Vblank from the display adjustment unit 70.

For example, in the display control process, even when the Equation (3) is satisfied, the display timing Vblank is adjusted to reduce the necessary duration Y as short as possible as long as the Equation (3) is satisfied.

For this reason, in the odd-numbered field image $n_{odd}$ and the even-numbered field image $n_{even}$, the time necessary from the write start time to the display timing is shortened as a whole. Therefore, it is possible to display an image with a minimum delay.

2. Second Embodiment

<Example When Display Timing Vblank Is Not Adjusted>

In the receiver 22, the display timing Vblank is adjusted based on a comparison result between the necessary duration Y and the allowable delay time Yth as illustrated in Figs. 7 and 8. Therefore, it is possible to suppress an image distortion and the like and display an image with a minimum delay.

Alternatively, it is possible to suppress an image distortion and the like, for example, without adjusting the display timing Vblank.

That is, in the receiver 22, it is possible to suppress an image distortion and the like if the Equation (3) is satisfied. Therefore, it is possible to display an image in synchronization with the original display timing Vblank without adjusting the display timing Vblank.

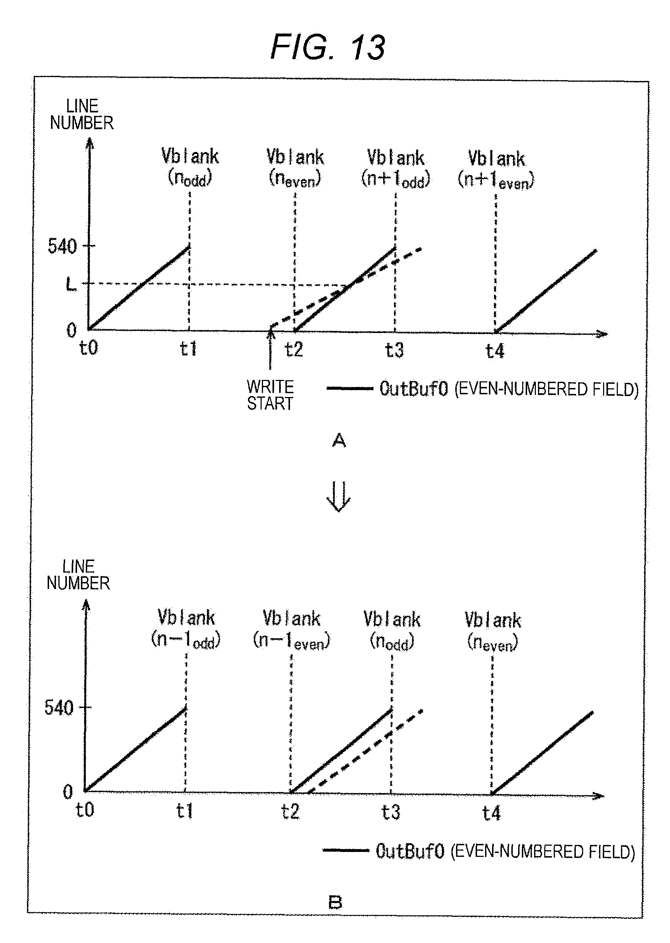

In a case where the Equation (3) is not satisfied, for example, the receiver 22 suppress an image distortion and the like by delaying the display timing of the image as illustrated in Fig. 13.

Fig. 13 illustrates an exemplary case where the image display timing is delayed in a case where the Equation (3) is not satisfied.

If the Equation (3) is not satisfied, as illustrated in Fig. 13A, the line(i) ($i \geq L$) of the line number i in the even-numbered field image $n_{even}$ is written to the buffer 65 after the line display timing for displaying the line(i) elapses.

In this case, if the even-numbered field image $n_{even}$ is displayed at the time point t2 as the display timing Vblank ($n_{even}$), a distortion and the like may be generated in the even-numbered field image $n_{even}$.

Therefore, the write control unit 64 performs control such that the even-numbered field image $n_{even}$ is written to the buffer 65 at the time point t4 after the time point t2 as illustrated in Fig. 13B.

In this case, it is assumed that the write control unit 64 has built-in memory (not illustrated), and the write timing to the buffer 65 is adjusted while the even-numbered field image $n_{even}$ from the decoding unit 63 is held in a built-in memory (not illustrated).

As a result, the display control unit 66 can display the even-numbered field image $n_{even}$ by setting the display timing Vblank($n_{even}$) to the time point t4 by shifting the display timing Vblank($n_{even}$) from the time point t2 to the time point t4.

2. Modifications

It may be possible to suppress an image distortion and the like by combining both a case where the display timing Vblank is adjusted and a case where the display timing Vblank is not adjusted.

Specifically, for example, if the Equation (3) is satisfied, the display timing Vblank may be adjusted as illustrated in Fig. 7. If the Equation (3) is not satisfied, the display timing Vblank may be shifted as illustrated in Fig. 13.

For example, if the Equation (3) is satisfied, an image may be displayed at the original display timing Vblank without adjustment. If the Equation (3) is not satisfied, the display timing Vblank may be adjusted as illustrated in Fig. 8.

According to the first and second embodiments, the necessary duration Y and the allowable delay time Yth are computed based on the write information of the even-numbered field image $n_{even}$. Alternatively, for example, as illustrated in Fig. 14, the necessary duration Y and the allowable delay time Yth may be computed based on the write information of the odd-numbered field image $n_{odd}$. Alternatively, for example, the necessary duration Y and the allowable delay time Yth may be computed based on both the write information of the odd-numbered field image $n_{odd}$ and the write information of the even-numbered field image $n_{even}$.

For example, in a case where an image is displayed on the display unit 67 in a progressive mode, each image is converted into a progressive image, and the necessary duration Y and the allowable delay time Yth are computed based on the write information of the progressive image as illustrated in Fig. 15.

When the X-distribution information indicating a distribution of the write preparation durations Xn of Fig. 4 is created, the display adjustment unit 70 creates the X-distribution information by setting a frequency of the write preparation durations Xn to 1 regardless of the measurement time of the write preparation duration Xn.

However, the display adjustment unit 70 may create the X-distribution information by increasing the frequency of the write preparation duration Xn for the more recent measurement time. It is conceived that this is because the more recently measured write preparation duration Xn more accurately represents the write preparation duration of the image received from that time.

The same applies to the case where the P-distribution information indicating a distribution of the buffer write durations Pn is created as illustrated in Fig. 5.

As a result, using the display adjustment unit 70, it is possible to create the X-distribution information by more accurately reflecting the write preparation duration of the image received from that time and create the P-distribution information by more accurately reflecting the buffer write duration of the image received from that time.

For this reason, the display adjustment unit 70 can more accurately compute the necessary duration Y and the allowable delay time Yth of the image received from that time based on the X-distribution information and the P-distribution information. Therefore, it is possible to more appropriately adjust the display timing Vblank depending on the received image.

The present technique may also be embodied as follows.

(1) A display control apparatus including: a receive unit that receives an image; a write unit that writes the image in a holding unit that temporarily holds the received image; a first computation unit that computes an allowable delay time indicating a delay time allowed when it is difficult to terminate the writing within a predetermined elapsed time from a write start time at which the image starts to be written; a second computation unit that computes a necessary duration that is necessary at least until a display timing, at which the image starts to be displayed, from the write start time of the image written within a delay time equal to or shorter than the allowable delay time; a comparison unit that compares the allowable delay time and the necessary duration; a display adjustment unit that adjusts the display timing based on a result of the comparison in the comparison unit; and a display control unit that displays the image in synchronization with the adjusted display timing.

(2) The display control apparatus according to the paragraph (1), further including a first measurement unit that measures a write time necessary to write the image, wherein the first computation unit computes the allowable delay time based on a distribution of the write time.

(3) The display control apparatus according to the paragraph (1) or (2), further including a second measurement unit that measures a write preparation duration necessary to start the write the image, wherein the second measurement unit computes the necessary duration based on the distribution of the write preparation duration.

(4) The display control apparatus according to any one of the paragraphs (1) to (3), wherein the display control terminates display of the image at a predetermined time from the display timing in synchronization with the adjusted display timing, and the display adjustment unit adjusts the display timing to shorten the necessary duration as long as a condition that the necessary duration is longer than the allowable delay time is satisfied in a case where the necessary duration is longer than the allowable delay time as a result of the comparison.

(5) The display control apparatus according to the paragraph (4), wherein the display adjustment unit adjusts the display timing to obtain the necessary duration longer than the allowable delay time in a case where the necessary duration is not longer than the allowable delay time as a result of the comparison.

(6) The display control apparatus according to claim (4), wherein the write unit starts to write the image after the first display timing in a case where the necessary duration is not longer than the delay time as a result of the comparison, and the display control unit displays the image in synchronization with a second display timing later than the first display timing.

(7) The display control apparatus according to any one of the paragraphs (1) to (3), wherein the display control unit displays the image written with a delay time equal to or shorter than the allowable delay time in synchronization with the adjusted display timing.

A series of the aforementioned processes may be executed using hardware or software. In a case where a series of processes are executed using software, a program embodied in the software is installed from a program recording medium, for example, to a computer integrated into dedicated hardware or a general-purpose computer capable of executing various functionalities by installing various programs.

<Exemplary Computer Configuration>

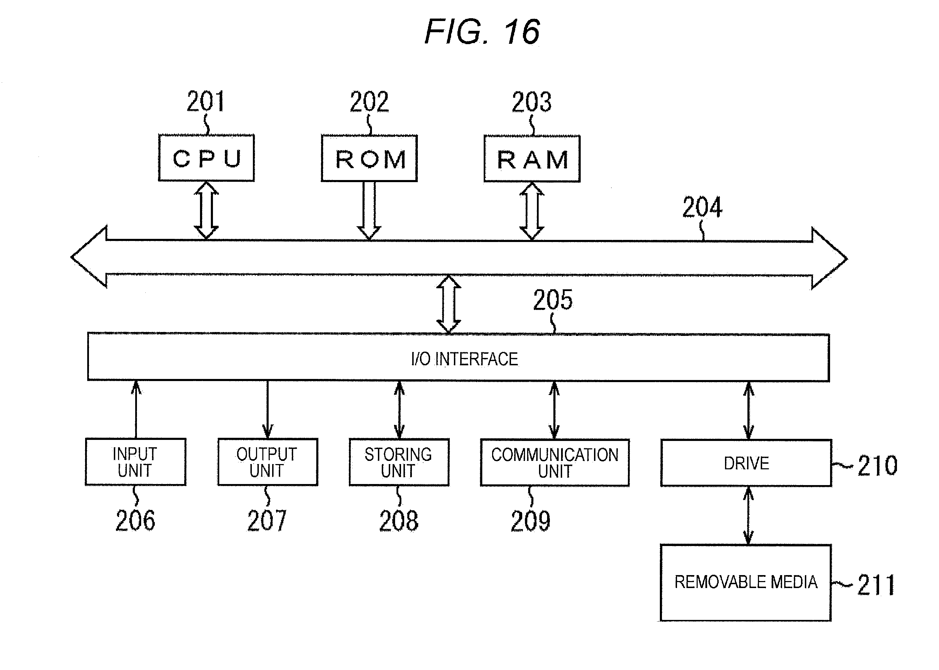

Fig. 16 is a block diagram illustrating an exemplary configuration of hardware of a computer that executes a series of the aforementioned processes using a program.

A central processing unit (CPU) 201 executes various processes depending on a program stored in a read-only memory (ROM) 202 or a storing unit 208. A random access memory (RAM) 203 appropriately stores a program or data executed by the CPU 201. The CPU 201, the ROM 202, and the RAM 203 are connected to each other via a bus 204.

An input/output (I/O) interface 205 is connected to the CPU 201 via a bus 204. An input unit 206 such as a keyboard, a mouse, and a microphone and an output unit 207 such as a loudspeaker are connected to the I/O interface 205. The CPU 201 executes various processes depending on an instruction input from the input unit 206. In addition the CPU 201 outputs the processing result to the output unit 207.

The storing unit 208 connected to the I/O interface 205 includes, for example, a hard disk and stores various data or programs executed by the CPU 201. A communication unit 209 communicates with an external device via a network such as the Internet or a local area network (LAN).

The program may be obtained using the communication unit 209 and may be stored in the storing unit 208.

When a remote medium such as a magnetic disc, an optical disc, an opto-magnetic disc, or a semiconductor memory is installed, the drive 210 connected to the I/O interface 205 drives the remote medium 211 to obtain a program, data, and the like recorded therein. The obtained program or data are transmitted to the storing unit 208 and stored therein as necessary.

A recording medium that records (stores) a program installed in a computer in an executable state includes a magnetic disc (such as a flexible disc), an optical disc (such as a compact disc-read only memory (CD-ROM) or a digital versatile disc (DVD)), an opto-magnetic disc (such as mini-disc (MD)), a remote medium 211 as a package medium such as a semiconductor memory, a ROM 202 where a program is stored temporarily or permanently, or a hard disk included in the storing unit 208 as illustrated in Fig. 16. A program is recorded in a recording medium via a wired/wireless communication medium such as a local area network (LAN), the Internet, a digital satellite broadcasting network using a communication unit 209 serving as an interface such as a router or modem as necessary.

In the present specification, steps that describe a series of the aforementioned processes may be performed in a time-series manner in the order of description or may be executed in parallel or individually without being necessarily processed in a time-series manner.

The embodiments of the present invention are not limited to the first and second embodiments described above. Instead, the invention may be variously changed or modified without departing from the spirit and scope of the invention.

REFERENCE SIGNS LIST

22 Receiver, 61 RTP receive unit, 62 Packet assembling unit, 63 Decoding unit, 64 Write control unit, 65 Buffer, 66 Display control unit, 67 Display unit, 68 Write information measurement unit, 69 Write information storing unit, 70 Display adjustment unit, 71 Operation unit, 72 Control unit

**Claims**

1.  A display control apparatus comprising:

    a receive unit that receives an image;
    a write unit that writes the image in a holding unit that temporarily holds the received image;
    a first computation unit that computes an allowable delay time indicating a delay time allowed when it is difficult to terminate the writing within a predetermined elapsed time from a write start time at which the image starts to be written;
    a second computation unit that computes a necessary duration that is necessary at least until a display timing, at which the image starts to be displayed, from the write start time of the image written within a delay time equal to or shorter than the allowable delay time;
a comparison unit that compares the allowable delay time and the necessary duration;
a display adjustment unit that adjusts the display timing based on a result of the comparison in the comparison unit; and
a display control unit that displays the image in synchronization with the adjusted display timing.

2. The display control apparatus according to claim 1, further comprising a first measurement unit that measures a write time necessary to write the image,
wherein the first computation unit computes the allowable delay time based on a distribution of the write time.

3. The display control apparatus according to claim 1, further comprising a second measurement unit that measures a write preparation duration necessary to start the write the image,
wherein the second measurement unit computes the necessary duration based on the distribution of the write preparation duration.

4. The display control apparatus according to claim 1, wherein the display control unit terminates display of the image at a predetermined time from the display timing in synchronization with the adjusted display timing, and
the display adjustment unit adjusts the display timing to shorten the necessary duration as long as a condition that the necessary duration is longer than the allowable delay time is satisfied in a case where the necessary duration is longer than the allowable delay time as a result of the comparison.

5. The display control apparatus according to claim 4, wherein the display adjustment unit adjusts the display timing to obtain the necessary duration longer than the allowable delay time in a case where the necessary duration is not longer than the allowable delay time as a result of the comparison.

6. The display control apparatus according to claim 4, wherein the write unit starts to write the image after the first display timing in a case where the necessary duration is not longer than the delay time as a result of the comparison, and
the display control unit displays the image in synchronization with a second display timing later than the first display timing.

7. The display control apparatus according to claim 1, wherein the display control unit displays the image written with a delay time equal to or shorter than the allowable delay time in synchronization with the adjusted display timing.

8. A display control method of a display control apparatus that displays an image, the display control method comprising:

   a receiving step of receiving the image;
   a writing step of writing the image to a holding unit that temporarily holds the received image;
   a fist computing step of computing an allowable delay time indicating a delay time allowable when it is difficult to terminate the writing within a predetermined elapsed time from a write start time at which the image starts to be written;
   a second computing step of computing a necessary duration that is necessary at least until a display timing, at which the image starts to be displayed, from the write start time of the image written within a delay time equal to or shorter than the allowable delay time;
   a comparing step of comparing the allowable delay time and the necessary duration;
   a display adjusting step of adjusting the display timing based on a result of the comparison in the comparing step; and
   a display controlling step of displaying the image in synchronization with the adjusted display timing.

9. A program causing a computer to serve as:

   a receiving control unit that receives an image;
   a write unit that writes the image in a holding unit that temporarily holds the received image;
   a first computation unit that computes an allowable delay time indicating a delay time allowed when it is difficult to terminate the writing within a predetermined elapsed time from a write start time at which the image starts to be written;
   a second computation unit that computes a necessary duration that is necessary at least until a display timing, at which the image starts to be displayed, from the write start time of the image written within a delay time equal to or shorter than the allowable delay time;
a comparison unit that compares the allowable delay time and the necessary duration;
a display adjustment unit that adjusts the display timing based on a result of the comparison in the comparison unit; and
a display control unit that displays the image in synchronization with the adjusted display timing.

## FIG. 1

EP 2 698 993 A1

EP 2 698 993 A1

## FIG. 2

## FIG. 3

FIG. 4

# FIG. 5

# FIG. 6

# FIG. 7

A

⇓

B

# FIG. 8

## FIG. 9

TIME

t3

t2 (START DISPLAY)

t1

t0 (START CAPTURING)

PACKET NUMBER

| Packet | Stages |
|---|---|
| 1 | CAPTURING / CODING / PACKET CREATING / TRANSMISSION / PACKET ASSEMBLING / DECODING |
| 2 | CAPTURING / CODING / PACKET CREATING / TRANSMISSION / PACKET ASSEMBLING / DECODING |
| 3 | CAPTURING / CODING / PACKET CREATING / TRANSMISSION / PACKET ASSEMBLING / DECODING |
| ... | ... |
| n | CAPTURING / CODING / PACKET CREATING / TRANSMISSION / PACKET ASSEMBLING / DECODING |

*FIG. 10*

# FIG. 11

```
          ┌─────────────────────────┐
          │  START DISPLAY CONTROL  │
          │        PROCESS          │
          └─────────────────────────┘
                      │
                      ▼
          ┌─────────────────────────┐  S21
          │      RECEIVE PACKET      │
          └─────────────────────────┘
                      │
                      ▼
          ┌─────────────────────────┐  S22
          │      ASSEMBLE PACKET     │
          └─────────────────────────┘
                      │
                      ▼
          ┌─────────────────────────┐  S23
          │     DECODE CODED DATA    │
          └─────────────────────────┘
                      │
                      ▼
          ┌─────────────────────────┐  S24
          │  WRITE IMAGE DATA OBTAINED │
          │  THROUGH DECODING TO BUFFER │
          └─────────────────────────┘
                      │
                      ▼
          ┌─────────────────────────┐  S25
          │   READ DATA FROM BUFFER AND DISPLAY │
          │  DATA AT DISPLAY TIMING ADJUSTED THROUGH │
          │   DISPLAY TIMING ADJUSTMENT PROCESS │
          └─────────────────────────┘
                      │
                      ▼
          ┌─────────────────────────┐  S26
          │ MEASURE AND STORE X-VALUE FOR EACH FIELD │
          └─────────────────────────┘
                      │
                      ▼
          ┌─────────────────────────┐  S27
          │ MEASURE AND STORE P-VALUE FOR EACH FIELD │
          └─────────────────────────┘
                      │
                      ▼
          < DOES PREDETERMINED TIME ELAPSE? >  S28
                      │              NO ──────►
                     YES
                      │
                      ▼
          ┌─────────────────────────┐  S29
          │ DISPLAY TIMING ADJUSTMENT PROCESS │
          └─────────────────────────┘
```

# FIG. 12

```
        ┌─────────────────────────────────────────┐
        │  START DISPLAY TIMING ADJUSTMENT PROCESS │
        └─────────────────────────────────────────┘
                          │
                          ▼
        ┌─────────────────────────────────────────┐  S41
        │      COMPUTE DISTRIBUTION OF WRITE       │
        │         PREPARATION DURATION X           │
        └─────────────────────────────────────────┘
                          │
                          ▼
        ┌─────────────────────────────────────────┐  S42
        │ COMPUTE THRESHOLD VALUE Xth BASED ON     │
        │ DISTRIBUTION OF WRITE PREPARATION        │
        │              DURATION X                  │
        └─────────────────────────────────────────┘
                          │
                          ▼
        ┌─────────────────────────────────────────┐  S43
        │ COMPUTE NECESSARY DURATION Y BASED ON    │
        │ THRESHOLD VALUE Xth AND DISPLAY INTERVAL │
        │            Vblank_Interval               │
        └─────────────────────────────────────────┘
                          │
                          ▼
        ┌─────────────────────────────────────────┐  S44
        │ COMPUTE DISTRIBUTION OF BUFFER WRITE     │
        │              DURATION P                  │
        └─────────────────────────────────────────┘
                          │
                          ▼
        ┌─────────────────────────────────────────┐  S45
        │ COMPUTE THRESHOLD VALUE Pth BASED        │
        │ ON DISTRIBUTION OF BUFFER WRITE TIME P   │
        └─────────────────────────────────────────┘
                          │
                          ▼
        ┌─────────────────────────────────────────┐  S46
        │ COMPUTE ALLOWABLE DELAY TIME Yth BASED ON│
        │ THRESHOLD VALUE Pth AND ANTICIPATED      │
        │           WRITE TIME 1/60                │
        └─────────────────────────────────────────┘
                          │
                          ▼
                  ┌───────────────┐  S47
                  │   Y > Yth?    │──── NO ────┐
                  └───────────────┘            │
                      │ YES                    │
                      ▼                        ▼
        ┌──────────────────┐  S48    ┌──────────────────┐  S49
        │  DELAY DISPLAY   │         │  DELAY DISPLAY   │
        │  TIMING BY -Z    │         │  TIMING BY Z     │
        └──────────────────┘         └──────────────────┘
                      │                        │
                      ▼◄───────────────────────┘
                ┌──────────┐
                │  RETURN  │
                └──────────┘
```

# FIG. 13

A

⇩

B

# FIG. 14

LINE
NUMBER

Vblank
$(n-1_{odd})$

Vblank Interval

Vblank
$(n_{odd})$

$Y_{th}\left(=P_{th}-\dfrac{1}{60}\right)$

Vblank
$(n-1_{even})$

$P_{th}$

540

$\dfrac{1}{60}$

ANTICIPATED
WRITE

WRITE DELAY

DISPLAY

0

TIME

$X_{th}$

$T_s$   Y

# FIG. 15

LINE
NUMBER

Vblank $(n-1)$          Vblank $(n)$

$Y_{th}\left(=P_{th}-\dfrac{1}{60}\right)$

Vblank Interval

$P_{th}$

540

$\dfrac{1}{60}$

ANTICIPATED
WRITE

WRITE DELAY

DISPLAY

0

TIME

$X_{th}$  $T_s$   Y

# FIG. 16

CPU 201

ROM 202

RAM 203

204

205

I/O INTERFACE

INPUT UNIT 206

OUTPUT UNIT 207

STORING UNIT 208

COMMUNICATION UNIT 209

DRIVE 210

REMOVABLE MEDIA 211

EP 2 698 993 A1

| INTERNATIONAL SEARCH REPORT | International application No. |
|---|---|
| | PCT/JP2012/058379 |

A. CLASSIFICATION OF SUBJECT MATTER
*H04N7/173(2011.01)i, H04L12/56(2006.01)i*

According to International Patent Classification (IPC) or to both national classification and IPC

B. FIELDS SEARCHED

Minimum documentation searched (classification system followed by classification symbols)
H04N7/173, H04L12/56

Documentation searched other than minimum documentation to the extent that such documents are included in the fields searched
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2009-278545 A  (Sony Corp.), 26 November 2009 (26.11.2009), paragraphs [0091] to [0093] & US 2009/0285310 A1 | 1-9 |
| A | JP 2010-35003 A  (NEC Corp.), 12 February 2010 (12.02.2010), paragraphs [0053] to [0070]; fig. 5 to 6 & US 2010/0027567 A1    & EP 2149999 A1 | 1-9 |
| A | JP 11-163892 A  (Matsushita Electric Industrial Co., Ltd.), 18 June 1999 (18.06.1999), paragraphs [0077] to [0079]; fig. 9 to 10 (Family: none) | 1-9 |

| ☒ | Further documents are listed in the continuation of Box C. | ☐ | See patent family annex. |
|---|---|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 11 May, 2012 (11.05.12) | 22 May, 2012 (22.05.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2012/058379 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 64-29141 A  (NEC Corp.),<br>31 January 1989 (31.01.1989),<br>page 3, lower part<br>(Family: none) | 1-9 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007311924 A